Europäisches Patentamt

European Patent Office

Office européen des brevets

(11) Numéro de publication: **0 205 358**
**A1**

## (12) DEMANDE DE BREVET EUROPEEN

(21) Numéro de dépôt: 86400850.3

(22) Date de dépôt: 21.04.86

(51) Int. Cl.4: **A61M 37/02**

(30) Priorité: 24.04.85 FR 8506516

(43) Date de publication de la demande:
17.12.86 Bulletin 86/51

(84) Etats contractants désignés:
AT BE CH DE FR GB IT LI LU NL SE

(71) Demandeur: **Lenck, Lucien Charles**
**34 rue Maréchal de Lattre de Tassigny**
**F-63000 Clermont-Ferrand(FR)**
Demandeur: **Université de**
**CLERMONT-FERRAND I**
**Etablissement à caractère scientifique et**
**culturel 49 boulevard Gergovia**
**F-63001 Clermont-Ferrand(FR)**

(72) Inventeur: **Lenck, Lucien Charles**
**34 rue Maréchal de Lattre de Tassigny**
**F-63000 Clermont-Ferrand(FR)**
Inventeur: **Université de CLERMONT-FERRAND**
**I**
**Etablissement à caractère scientifique et**
**culturel 49 boulevard Gergovia**
**F-63001 Clermont-Ferrand(FR)**

(74) Mandataire: **Chanet, Jacques**
**Conseil en Brevets 129 Avenue de Royat**
**B.P. 27**
**F-63400 Chamalières(FR)**

(54) **Méthode et dispositif de fécondation "in vivo-in vitro".**

(57) La présente invention est du domaine de la fécondation artificielle.

Un dispositif destiné à mette en oeuvre une méthode de fécondation "in vitro -in vivo" consistant essentiellement à prélever sur la mère un ovocyte dans son follicule, à perforer la paroi utérine de la mère porteuse, à placerdans la perforation de cette paroi une capacité de culture, à introduire dans ladite capacité l'ovocyte et son liquide folliculaire, les spermatozoïdes et un milieu de culture, de telle sorte qu'après fécondation et multplication des cellules dans des conditions artificielles optimales, le transfert de l'embryon et la nidation puissent s'effectuer de façon sensiblement naturelle, est principalement caractérisé en ce que ladite capacité a la forme d'un récipient 1 constitué d'un col 2 assez rigide d'une longeur sensiblement égale à l'épaisseur de la paroi utérine, ledit col étant pourvu au moins extérieurement d'un relief 8 destiné à éviter le glissement du col le long de la perforation de la paroi 12 et d'une partie 3 dite ballon à paroi souple constituant la capacité proprement dite.

fig.1 .

## "CAPACITE TRANS-UTERINE POUR FECONDATION IN VITRO IN VIVO"

La présente invention est du domaine des techniques médicales visant à remédier à certains cas de stérilité, des êtres humains notamment, et elle a plus particulièrement pour objet un dispositif permettant de pratiquer la fécondation artificielle.

On rappelle que l'on entend par fécondation artificielle la technique permettant la fécondation de l'ovule (ou ovocyte) par le spermatozoïde dans des conditions qui ne sont pas celles prévues par la nature, par exemple les conditions dite "in-vitro". Une technique de fécondation in-vitro consiste à prélever l'ovule dans son follicule sous contrôle coelioscopique ou échographique, à laisser féconder après capacitation des spermatozoïdes, à laisser se multiplier dans un milieu physiologique pendant près de quarante-huit heures, à injecter enfin l'embryon formé dans l'utérus de la mère porteuse en espérant son implantation, ou nidation.

Les étapes in-vitro de l'opération sus-décrite sont réalisées en laboratoire dans des conditions les plus proches possibles de celles du milieu naturel de la fécondation c'est-à-dire la cavité péritonéale.

La méthode sus-visée, bien qu'ayant le mérite de remédier à certains cas de stérilité, présente toutefois un taux de réussite assez limité, de l'ordre de 10 à 25 %. Cette faible fiabilité paraît principalement due à la difficulté de la dernière étape, c'est-à-dire de la réimplantation de l'oeuf fécondé, ou embryon. Une des raisons de cette difficulté pourrait résider dans la fermeture du col utérin, par lequel doit être introduite la canule d'implantation de l'oeuf, trois jours après l'ovulation ; ce dernier délai résulte des durées cumulées de fécondation et de multiplication in-vitro. Or, la pénétration du col utérin, fermé à cette période post-ovulatoire, se révèle souvent hémorragique, et il est connu que le sang est un facteur défavorable à l'implantation de l'oeuf.

D'autres raisons pourraient expliquer le faible taux de fiabilité : on sait qu'il existe normalement une pression négative de quelques millimètres de mercure, par rapport à la pression atmosphérique, dans la cavité péritonéale ; cette dépression est normalement transmise dans la cavité utérine par les trompes de Fallope normalement perméables. Or, de nombreux cas de stérilité sont dus précisément à l'obturation des trompes. Dans la méthode sus-décrite de fécondation in-vitro, l'injection de liquide dans l'utérus entraînerait plutôt une surpression, laquelle pourrait nuire au bon contact entre l'oeuf et la muqueuse utérine.

Certaines stérilités traitées par fécondation in-vitro se présentent cependant malgré des trompes perméables ; le jeu précité des pressions pourrait alors expliquer que l'on retrouve dans les trompes l'embryon en phase de croissance : l'oeuf aurait été aspiré sous l'effet des pressions positives intra-utérines vers les pressions négatives intra-abdominales, ou péritonéales.

La présente invention, dont la méthode de fécondation va être exposée ci-après, vise entre autre ce double objectif , d'opérer le transfert sans avoir à forcer le passage utérin, donc en évitant l'hémorragie, de laisser s'exercer, voire de favoriser, la dépression péritonéale dans la cavité utérine, et de ne nécessiter que des moyens simples de mise en oeuvre.

La méthode de l'invention dite de fécondation "in vitro-in vivo" consiste essentiellement à prélever sur la mère un ovocyte dans son follicule, à perforer une paroi de la mère porteuse, ladite paroi séparant le milieu intrapéritonéal du milieu extrapéritonéal, à placer dans la perforation de cette paroi une capacité de culture, à introduire dans ladite capacité l'ovocyte et son liquide folliculaire, les spermatozoïdes et un milieu de culture, de telle sorte qu'après fécondation et multiplication des cellules dans des conditions artificielles optimales, le transfert de l'embryon et la nidation puissent s'effectuer de façon sensiblement naturelle.

Il apparaît déjà, à l'examen de cette méthode, que l'introduction, éventuellement hémorragique au passage du col utérin, de l'oeuf dans la capacité de culture précède d'un délai supérieur à au moins trois jours, le transfert de l'embryon dans la cavité utérine, et que ce transfert s'effectue dans des conditions plus proches des conditions naturelles que sont celles du transfert depuis l'ovaire à travers les trompes jusqu'à la cavité utérine.

La méthode sus-définie est mise en oeuvre de façon optimale grâce à un dispositif à fonction de récipient constituant ladite capacité, ledit récipient étant constitué, de façon caractéristique, d'un col assez rigide d'une longueur sensiblement égale à l'épaisseur de la paroi utérine, ledit col étant pourvu au moins extérieurement d'un relief destiné à éviter le glissement du col le long de la perforation de la paroi, et d'une partie dite ballon constituant la capacité proprement dite ; de préférence le relief est un relief circonférentiel simple disposé dans un plan sensiblement perpendiculaire à l'axe du col.

Suivant une autre caractéristique principale de l'invention la partie dite ballon est constituée d'une matière souple. Cette dernière caractéristique permet, entre autre chose, la transmission dans la cavité utérine de la dépression péritonéale.

Avantageusement, les matières constitutives du col et du ballon définies ci-dessus comme assez rigide pour l'un et souple pour l'autre, sont en outre l'une et l'autre élastiques.

De préférence la matière constitutive du col est un élastomère de silicone à dureté maximale, tandis que la matière constitutive du ballon est un élastomère de silicone à assez faible dureté mais à forte élasticité.

De préférence encore, l'extrémité du col opposé au ballon est conformée en collerette à secteurs et avantageusement chaque secteur comporte une rainure de pliage. L'intérêt de la collerette est d'éviter l'obturation de l'orifice du récipient par un développement de la muqueuse utérine, et cela au moins pendant le délai estimé du transfert.

Suivant une forme préférée de réalisation la forme du col est celle de la surface de deux segments ellipsoïdaux (tonnelets) coaxiaux, la partie de jonction des segments formant en creux ledit relief ; grâce à cette disposition le muscle constituant la paroi péritonéale peut trouver un appui ferme dans le relief en creux tandis que la paroi intérieure du col qui présente en plein un relief correspondant favorise la rétention d'un organe provisoire de bouchage, comme cela sera expliqué ultérieurement.

De préférence enfin, un crin dit de guidage, est attaché à l'intérieur du col, de telle manière qu'au moyen de mouvements de torsion exercés sur le crin, l'attache puisse se rompre.

La présente invention sera mieux comprise, et des détails en relevant apparaîtront à la description qui va être faite tant de la méthode que du dispositif, en relation avec les figures de la planche unique annexée dans laquelle :

-la fig.1 est une illustration en perspective et à l'échelle agrandie d'une capacité permettant la mise en oeuvre de la méthode de l'invention,

-la fig.2 est une illustration conventionnelle en coupe verticale d'organes génitaux dans lesquels a été implanté un dispositif de la fig.1,

- la fig.3 est une coupe illustrative des moyens d'implantation du dispositif des figures précédentes,

-la fig.4 représente une autre forme de réalisation de la capacité de l'invention, et

-la fig.5 illustre une implantation de la capacité de la figure précédente, sur la mère perteuse.

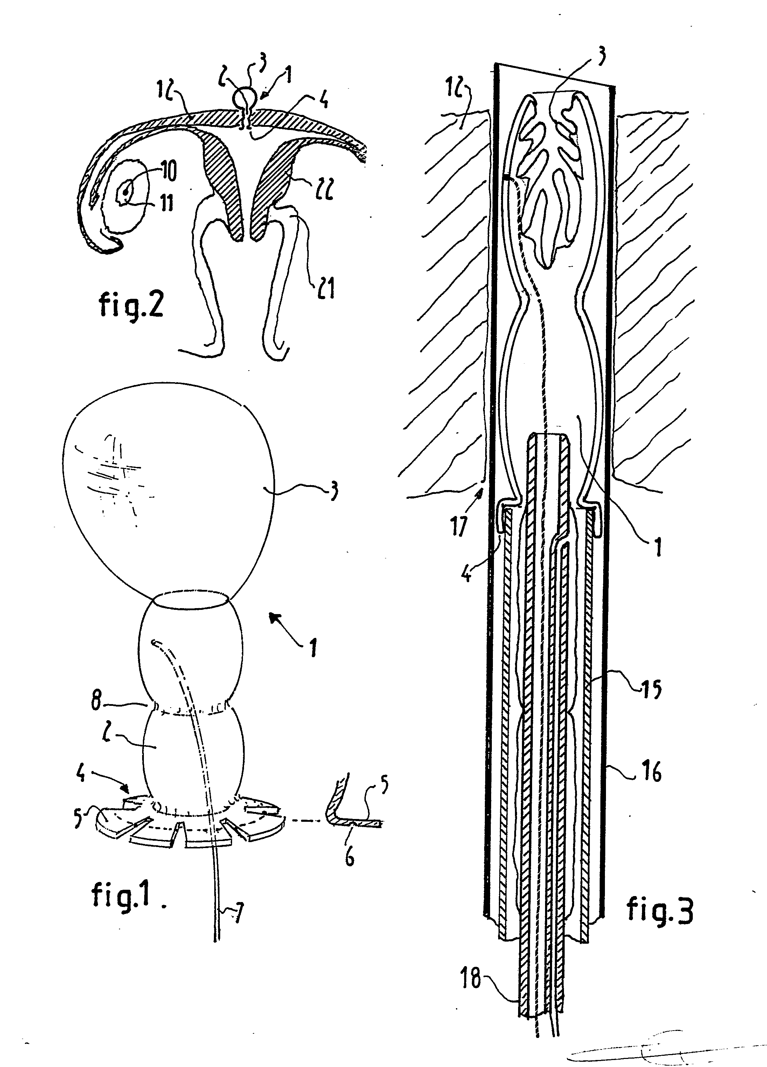

Sur la fig.1, un dispositif 1 conforme à l'invention a la structure générale d'un récipient constitué d'un col 2 et d'une capacité 3, dite ballon, approximativement sphérique ; la matière constitutive de la paroi du ballon est un élastomère de silicone, de préférence à d'autres élastomères tels que le caoutchouc naturel, les élastomères de polyester, etc... en raison de la perméabilité du silicone au gaz carbonique et en raison de l'inertie chimique de cette matière ; le col 2 est réalisé en un matière, toujours élastique de préférence, mais plus rigide que la matière constitutive du ballon ; la matière constitutive du col pourra être par exemple un élastomère de silicone d'assez grande dureté - (70°Shore) ; la structure du col pourra être encore composite, par exemple constituée d'une armature tubulaire de forme identique en polycarbonate et, revêtue intérieurement et extérieurement d'élastomère silicone.

L'extrémité du col opposée à celle comportant le ballon, est pourvue d'une collerette 4 formée de préférence d'une pluralité de secteurs tels que 5 légèrement espacés les uns des autres, chaque secteur comportant sur sa face inférieure une rainure de pliage 6. L'utilité de la conformation en secteurs de la rainure est de permettre le repliage de celle-ci pour permettre la pose du dispositif au moyen de tubulures, tel que cela sera décrit plus loin.

Toujours sur la fig.1, il apparaît qu'un crin de guidage 7 est relié à la face interne du col ; cette relation, ou attache, est suffisamment fragile pour pouvoir être rompue par torsion du crin ; cette attache sera avantageusement réalisée par collage de l'extrémité du crin à l'aide d'élastomère silicone ; l'utilité du crin de guidage sera elle aussi décrite plus loin.

Toujours sur la fig.1, on remarque encore la forme du col en tonnelets superposés (segments ellipsoïdaux coaxiaux). La longueur du col est de l'ordre de 15 à 20 millimètres c'est-à-dire légèrement supérieure à la paroi de fond, dit mur utérin, de la cavité utérine. Ainsi grâce au relief en creux 8 situé à la jonction des segments ellipsoïdaux, le muscle utérin pourra faire prise sur le col du dispositif.

Sur la fig.2 un dispositif tel que celui de la figure précédente a été placé dans une perforation de la paroi utérine en regard du col de l'utérus ; la collerette 4 est située dans la cavité utérine, tandis que le ballon 3 est logé dans la cavité péritonéale ; cette opération a pu précéder d'un certain temps la période d'ovulation. Lors de l'ovulation un ovocyte 10 est prélevé avec son liquide folliculaire 11 puis il est déposé au moyen d'une sonde appropriée dans le ballon dans lequel a préalablement été introduit un milieu physiologique ; l'introduction de l'ovule est immédiatement suivie d'une injection de spermatozoïdes au moyen d'une sonde analogue. La fécondation peut alors avoir lieu et la nidation s'opérer dans les jours consécutifs, nettement après la cessation de l'éventuelle hémorragie.

Sur la fig.3, on a illustré un instrument d'implantation du dispositif de l'invention, et en faisant référence à cette figure on va décrire un processus opératoire. Après repérage du fond utérin au moyen de la tubulure 16 dite externe, le mur utérin est perforé en 17 au moyen d'un trocart retiré à la fin du geste de perforation. Puis une tubulure interne 15 surmontée du dispositif 1, telle qu'elle apparaît sur la figure, est introduite dans la tubulure externe jusqu'à ce que le dispositif 1 soit positionné dans l'épaisseur de la perforation. La tubulure 16 est alors retirée ; on peut alors affiner par manipulation de la tubulure 15 la relation entre la collerette 4 du dispositif et la muqueuse utérine. A la fin de cette opération la tubulure interne est retirée à son tour.

Ultérieurement on introduit dans le col du dispositif l'extrémité distale d'une sonde valve 18 - (telle que de Folley ou de Tremann) en se guidant sur le crin de guidage 7 préalablement enfilé dans la lumière de la sonde. Lorsque l'extrémité de la sonde parvient en butée contre le point d'attache du crin, celui-ci est détaché du dispositif 1 par torsion sur lui-même ; la position de la sonde valve peut alors être réajustée dans le dispositif. Le ballonnet de la sonde valve est alors gonflé afin de créer un espace clos dont le remplissage permettra l'expulsion du ballon 3 hors du col 2 du dispositif et son expansion dans la cavité péritonéale. On aura ainsi créé la capacité pouvant recevoir le milieu et les éléments de fécondation.

Il est également prévu que l'extrémité inférieure du col 2 puisse être pourvue d'un clapet, par exemple fine lamelle d'élastomère afin d'éviter la remontée dans le ballon de l'embryon en cours de nidation.

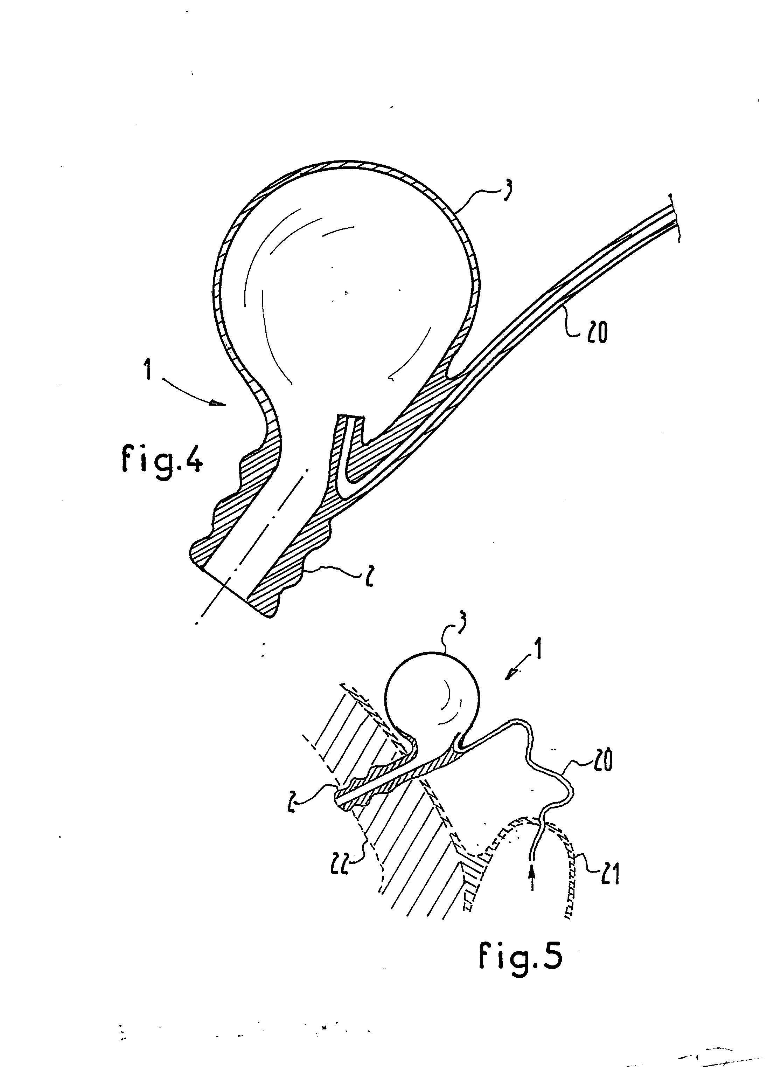

Sur la fig.4 une capacité ou récipient analogue à celle de la fig.1 s'en différencie cependant par la présence d'un tube souple 20 débouchant dans le ballonnet 3 au voisinage du col 2 ; la partie du tube 20 proche du col 2 a de préférence une rigidité voisine de celle du col 2.

Sur la fig.5 une capacité analogue à celle de la fig.4 est implantée dans la paroi utérine au voisinage du cul-de-sac de Douglas tandis que le tube 20 passe à travers une perforation de la paroi vaginale 21 ; le tube 20 permet une introduction dans la capacité soit des gamètes (ovules, spermatozoïdes) soit du milieu de culture, soit d'agents destinés à modifier ces derniers.

Bien que l'on ait décrit et représenté une forme particulière de réalisation d'un dispositif conforme à la présente invention, il doit être compris que la portée de cette dernière ne peut se limiter à cette forme mais qu'elle s'étend à tous les dispositifs comportant des caractéristiques générales énoncées plus haut.

## Revendications

1.-Méthode de fécondation dite "in vitro-in vivo" consistant à prélever sur la mère un ovocyte dans son follicule, à perforer une paroi de la mère porteuse, ladite paroi séparant le milieu intrapéritonéal du milieu extrapéritonéal, à placer dans la perforation de cette paroi une capacité de culture, à introduire dans ladite capacité l'ovocyte et son liquide folliculaire, les spermatozoïdes et un milieu de culture, de telle sorte qu'après fécondation et multiplication des cellules dans des conditions artificielles optimales, le transfert de l'embryon et la nidation puissent s'effectuer de façon sensiblement naturelle ;

2.-Méthode selon la revendication 1, caractérisée :

en ce que ladite paroi intrapéritonéale est la paroi utérine ;

3.-Méthode selon la revendication 1, caractérisé :

en ce que ladite paroi intrapéritonéale est la paroi vaginale ;

4.-Dispositif destiné à la mise en oeuvre de la méthode selon la revendication 1, caractérisé :

en ce que ladite capacité a la forme d'un récipient (1) constitué d'un col (2) assez rigide d'une longueur sensiblement égale à l'épaisseur de la paroi utérine, ledit col étant pourvu au moins extérieurement d'un relief (8) destiné à éviter le glissement du col le long de la perforation de la paroi (12) et d'une partie (3) dite ballon constituant la capacité proprement dite ;

5.-Dispositif selon la revendication 4, caractérisé :

en ce que la paroi de la partie dite ballon, est constitué d'une matière souple ;

6.-Dispositif selon la revendication 5, caractérisé :

en ce que les matières constitutives du col (2) et du ballon (3) sont élastiques ;

7.-Dispositif selon la revendication 6, caractérisé :

en ce que l'extrémité du col opposée au ballon est conformée en collerette (4) ;

8.-Dispositif selon la revendication 7, caractérisé :

en ce que ladite collerette est conformée en secteurs ;

9.-Dispositif selon la revendication 8, caractérisé :

en ce que chaque secteur (5) comporte une rainure (6) de pliage ;

10.-Dispositif selon la revendication 9, caractérisé :

en ce que la forme du col est celle de la surface de deux segments ellipsoïdaux (tonnelets) coaxiaux, la partie de jonction des segments formant en creux ledit relief (8) ;

11.-Dispositif selon la revendication 10, caractérisé :

en ce que la matière constitutive du col est un élastomère de silicone à dureté maximale, tandis que la matière constitutive du ballon est un élastomère de silicone à assez faible dureté mais à forte élasticité ;

12.-Dispositif selon la revendication 4, caractérisé :

en ce qu'un crin (7), dit de guidage, est attaché à l'intérieur du col (2), de telle manière qu'au moyen de mouvements de torsion exercés sur le crin, l'attache puisse se rompre ;

13.-Dispositif selon la revendication 4, caractérisé :

en ce que l'extrémité inférieure du col (2) est pourvue d'une membrane à fonction de clapet ;

14.-Dispositif selon la revendication 4, caractérisé :

en ce qu'un tube mince (20) débouche dans ladite capacité, ledit tube mince étant d'une assez grande longueur.

fig.2

fig.1 .

fig.3

fig.4

fig.5

**Office européen des brevets**

**RAPPORT PARTIEL DE RECHERCHE EUROPEENNE**

qui selon la règle 45 de la Convention sur le brevet européen est considéré, aux fins de la procédure ultérieure, comme le rapport de recherche européenne

Numéro de la demande

EP 86 40 0850

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| | La recherche n'a pas permis de relever des documents. | | A 61 M 37/02 |

**DOMAINES TECHNIQUES RECHERCHES (Int. Cl.4)**

A 61 M
A 61 D

### RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes: 4-14

Revendications ayant fait l'objet de recherches incomplètes:

Revendications n'ayant pas fait l'objet de recherches: 1-3

Raison pour la limitation de la recherche:

Méthode de traitement chirurgical ou thérapeutique du corps humain ou animal (voir art. 52(4) de la Convention sur le brevet européen).

| Lieu de la recherche | Date d'achèvement de la recherche | Examinateur |
|---|---|---|
| La Haye | 04-07-1986 | VANRUNXT |